# EUROPÄISCHE PATENTANMELDUNG

(11) **EP 2 158 931 A2**
(43) Veröffentlichungstag der Anmeldung: **03.03.2010**
(21) Anmeldenummer: 09075404.5
(22) Anmeldetag: 31.08.2009
(51) Int. Cl.: A61M 16/10, A61M 21/00

(54) **Sauerstoff-Klang-Therapie (S-K-T)**

(30) Priorität: 02.09.2008 DE 102008045648
(71) Anmelder: Waltraut Rudolph, 01723 Grumbach (DE)
(72) Erfinder: Waltraut Rudolph, 01723 Grumbach (DE)
(74) Vertreter: Boeckh, Tobias

(57) **Zusammenfassung**

Es wird ein Verfahren zur Entspannung und zur Reduzierung von Ermüdungserscheinungen vorgeschlagen, wobei Schallwellen durch klangerzeugende Elemente auf den Körper der zu behandelnden Person appliziert werden und zusätzlich reiner Sauerstoff oder ein Gasgemisch mit einem Sauerstoffanteil von über 90% Vol. mittels einer geeigneten Vorrichtung wie beispielsweise einer Atemmaske appliziert wird.

## Beschreibung

Die Erfindung betrifft ein Verfahren zur Entspannung und Reduzierung von Ermüdungserscheinungen und zur Behandlung von Stresszuständen körperlicher und seelischer Natur. Das erfindungsgemäße Verfahren ist beispielsweise zur Entgiftung des Körpers, zur Regeneration des Körpers nach Krankheiten bis hin in den kosmetischen Bereich anwendbar.

Im Stand der Technik sind Klangschalen (KS) bekannt. Es handelt sich hierbei vor allem um Schalen aus Bronze, die einen Ton erzeugen, wenn sie angeschlagen oder angerieben werden. Der Querschnitt der Klangschalen liegt im Bereich zwischen halbkugelförmig bis gongartig, mit einem mehr oder weniger flachen Boden, beziehungsweise mehr oder weniger nach innen geneigten Rändern. Der Durchmesser der Klangschalen des Standes der Technik variiert zwischen 10 und über 50 cm, wobei die Wanddicke zwischen 0,1 und 7mm variieren kann. Die Klangschalen stammen ursprünglich vornehmlich aus dem Raum Tibet, Nepal, China, Japan und Indien. Die Klangschalen sind in diesen Ländern in verschiedenen Ritualen verankert.

In der DE 199 51 943 ist ein Musikinstrument für die Klang- und Musiktherapie offenbart, mit welchem variable Klangräume ausgebildet werden können. Diese Patentschrift löst die Aufgabe, Resonanzkörper mit veränderlicher Größe und Form bereit zu stellen.

Die DE 20 2005 020 979 U1 beschreibt ein Instrument zur Klangtherapie mit welchem akustische Schwingungen auf einen menschlichen oder tierischen Körper übertragen werden. Die DE 20 2005 020 979 löst das Problem der Übertragung der Schwingungen durch einen Schwinger, der im Wesentlichen punktförmig mit einer Schwingungsbrücke verbunden ist, die eine freie Fläche zur Aufnahme von Schwingungen vom Musikinstrumenten oder einer anderen Schwingungsquelle aufweist.

Die DE 299 01 078 U1 offenbart eine Vorrichtung zur Klangtherapie mit mindestens einem in einem Gehäuse schwingend gelagerten und von diesen schwingungsmäßig entkoppelnden Schwingkörper, der durch eine an einer Gehäuseaußenseite vorgesehene Streich- und/oder Zupfseite in eine Schwingung versetzbar ist und Kontaktmittel aufweist, die zum Übertragen der Schwingung auf einem Rücken und/oder Kopfbereich eines im oder auf dem Gehäuse befindlichen Patienten ausgebildet sind.

Ein Musikinstrument für die Klang- beziehungsweise Musiktherapie wird in der DE 29 71 9376 U1 erörtert, bei welchem ein Grundkörper einen offenen oder halboffenen Raumkörper aufweist, der so ausgebildet ist, dass er wenigstens einen Spieler, einen Benutzer oder die zu behandelnde Person aufnehmen kann.

Eine Behandlungsliege zur Klangtherapie, die aus einem Klangkörper daran gespannter Saite beziehungsweise Saiten besteht, wobei der Klangkörper eine Größe und Form aufweist, dass eine Mensch bequem darauf sitzen oder liegen kann, beschreibt die DE 3925454.

Im Stand der Technik sind weiterhin mehrere Sauerstofftherapien beschrieben, so zum Beispiel die Sauerstoff-Langzeittherapie, die hyperbare Oxigenierung, die Sauerstoff-Mehrschritt-Therapie oder aber die Sauerstoffdusche. Die bekannteste Sauerstofftherapie ist die Sauerstoff-Mehrschritt-Therapie, bei der Sauerstoff inhaliert wird. Sie wurde von dem Physiker und Mediziner Manfred von Ardenne ab 1970 in Dresden entwickelt. Die Sauerstoff-Mehrschritt-Therapie wird in mehr als 20 Varianten durchgeführt und besteht in der Regel aus drei Schritten: Zunächst erhalten die Patienten ein Mineral/Vitamin-Cocktail. Im Anschluss daran inhalieren die Patienten Sauerstoff angereicherte Luft, danach beziehungsweise auch während des zweiten Schrittes betätigt sich der zu behandelnde Patient sportlich.

Die DE 698 24 752 T2 beschreibt eine Vorrichtung für die Sauerstofftherapie, welche eine pneumatisch betriebene und betätigte Einsparvorrichtung für die Zufuhr von atembaren Gas zu einem Patienten bereitstellt.

Nachteilhafterweise sind die offenbarten Verfahren nicht geeignet Stresszustände effizient und ohne Nebenwirkungen zu behandeln.

Insbesondere ist es mit den bekannten Verfahren nicht möglich, die Gehirnfunktion zu aktivieren, das Immunsystem stärken, eine effektive Erholung und Regeneration nach Krankheiten und deren oft beanspruchenden Behandlungen zu ermöglichen oder aber die Leber zu entgiften, die Zellsäuerung zu verringern und Menschen bei Stimmungsschwankungen zu unterstützen.

Es war völlig überraschend, dass eine Kombination von Klang- und Sauerstofftherapie ein neues und verbessertes Verfahren bereitstellt, welches die Nachteile des Standes der Technik nicht aufweist. Durch die Kombination von Klangschalentherapie und Sauerstofftherapie wird eine Verfahren zur Behandlung von Erschöpfungszuständen und Sauerstoffstress bereit gestellt, wobei Schallwellen durch klangerzeugende Elemente auf den Körper einer zu behandelnden Person appliziert werden und zusätzlich reiner Sauerstoff oder ein Gasgemisch mit einem Sauerstoffanteil mit über 90% Vol. mittels geeigneter Vorrichtungen wie beispielsweise eine Atemmaske appliziert werden. Der Stand der Technik legt es nicht nahe, diese beiden Therapien in der beschriebenen Weise miteinander zu kombinieren.

Die Schwingungen der KS, Gongs, Zimbel und Glocke versetzen den gesamten Körper in natürliche Schwingungen, um jede Zelle zu reinigen, regenerieren und erneuern. Die Wirkung ist ähnlich wie bei Ultraschallgerät. Verschiedene Frequenzen in unseren Zellen erzeugen Schwingungen, die durch die Klangtherapie aktiviert und harmonisiert werden. Angestaute Energien (Blockaden) werden aufgelöst und damit verbundene Beschwerden aufgelöst. Der natürliche Energiefluss kommt wieder ins fließen. Blockaden entstehen natürlich durch alltägliche Belastung und betreffen somit jeden Menschen. Es handelt sich hierbei nicht um ein Krankheitsbild, sondern vielmehr um einen Normalzustand der durch die Erfindung verbessert bzw. verändert werden kann.

Der Ablauf der Klangschalentherapie in Verbindung mit reinem Sauerstoff verbessert das Verfahren erheblich, da durch die Schwingungen der Sauerstoff besser aufgenommen werden kann und alle Zellen optimal mit Sauerstoff versorgt werden. Dies ist auch bei Menschen mit eingeschränkter Beweglichkeit anwendbar, im Gegensatz zu herkömmlichen Therapieverfahren (Sauerstoff- Mehrschritt - Therapie). Eine eingeschränkte Beweglichkeit im Sinne der Erfindung kann altersbedingt sein oder aber durch körperliche Belastung entstanden sein. Auch möglich ist eine eingeschränkte Beweglichkeit aufgrund von Bewegungsmangel oder Übergewicht.

Das Verfahren zeigt keinerlei Nebenwirkungen und ist bei allen Menschen anwendbar um das körperliche, seelische und geistige Befinden zu verbessern. Der Alterungsprozess wird auf natürliche Weise verlangsamt und eine deutliche Verbesserung der Lebensqualität erreicht. Sauerstoff und Energie im richtigen Verhältnis(im Energiefluss) sind lebensnotwendig um Organfunktionen aufrecht zu erhalten.

Es war völlig überraschend, dass das erfindungsgemäße Verfahren eingesetzt werden kann, um Vitalität und Selbstheilungskräfte der Patienten auf natürliche Weise ohne Nebenwirkungen zu aktivieren, wodurch dieses Verfahren zur Therapie und zur Steigerung der Leistungsfähigkeit eingesetzt werden kann. Wirkungen dieser Therapie können beispielsweise die Steigerung der Lebenserwartung sein, aber auch der Stressabbau, die Aktivierung der Gehirnfunktion wie Gedächtnis und Konzentration, die Anregung des Immunsystems, die Verbesserung des Kreislaufssystems, eine schnellere Erholung nach Krebsnachbehandlung, die Anhebung des Energiehaushalts und die Verringerung der Zellübersäuerung zur Unterstützung des seelischen Gleichgewichts und die Leberentgiftung.

Die Sauerstoff-Klang-Therapie ist besonders gut einsetzbar in Kureinrichtungen, Krankenhäusern, Rehabilitationskliniken, Wellness-Einrichtungen, Physiotherapien, Sportstudios, Hochleistungssportzentren, Alters- und Seniorenheimen, Suchtkliniken, im Hotelwesen u. a.

Besonders wirksam ist diese Therapie auch zur Leistungssteigerung bei Personen mit hohen Anforderungen im Alltag, z.B. Leistungssportler, Schauspieler, Manager usw.. Es wird die Hirnfunktion aktiviert, die Konzentration und Denkleistung deutlich verbessert und die Energie gesteigert. Durch die Verbesserung der Denkleistungen und der Konzentration sind auch Fortschritte bei Menschen mit Parkinson, Alzheimererkrankungen u. ä. Hirnleistungsstörungen erkennbar, da die gesunden Teile des Körpers gestärkt werden. Es ist mittlerweile bekannt, dass der Lebenswandel einen erheblichen Einfluss auf die Ausprägung der Krankheitssymptome hat. So kann beispielsweise das Gehirn eines Alzheimer-Patienten eine deutliche Ausprägung der Krankheit zeigen, ohne dass der Patient Symptome der Demenz aufweist. Diese Erkenntnis zeigt, dass der menschliche Körper in hohem Maße dazu in der Lage ist, Krankheiten bzw. deren Auswirkungen und Symptome zu kompensieren, wenn er entsprechend trainiert und gestärkt wurde. Das erfindungsgemäße Verfahren ist daher besonders gut geeignet um durch Stärkung des Körpers die Symptome von verschiedenen Krankheiten zu unterdrücken. Muskelzerrungen, Verspannungen, Ischiasbeschwerden, beispielsweise nach sportlicher Belastung sind ebenfalls effizient mit dieser Therapie zu beheben.

Die natürliche Heilung des Körpers von verschiedenen Krankheiten, z.B. Krebs, kann durch das Verfahren aktiviert werden. Hierdurch verbessert sich die Möglichkeit der Selbstheilung von Krebs ohne schädliche Nebenwirkungen.

Überraschenderweise eignen sich künstlich erzeugte Schwingungen weniger als Schallwellen, die mit klangerzeugenden Elementen wie Klangschalen oder Gongs generiert oder erzeugt werden.

In einer bevorzugten Ausführungsform der Erfindung wird der Sauerstoff oder das Gasgemisch mit einem Sauerstoffanteil von über 90% Vol. vor und/oder während und/oder nach der Schallwellenapplikation verabreicht. Jede dieser Vorzugsvarianten ist bei bestimmten Erschöpfungszuständen besonders gut anwendbar.

In einer weiteren bevorzugten Ausführungsform der Erfindung ist vorgesehen, dass als Elemente zur Klangerzeugung Klangschalen, Gongs, Zimbeln und/oder Glocken zum Einsatz kommen.

Die Klangschalen geben Energie und Klang nach außen oder nach innen. Klangschalen, welche nach innen schwingen, sind besonders für die Körperarbeit und Heilzwecke bevorzugt. Das Grundmaterial der Klangschalen ist Kupfer und Zinn. Weitere bevorzugte Metalle sind Silber, Quecksilber, Kupfer, Eisen und Blei. Bevorzugt variiert der Gewichtsprozentbereich von Zinn zwischen 20 und 25 Gew.-%. Bevorzugt weisen die Klangschalen insbesondere Kupfer, in kleineren Mengen Zinn, gefolgt von Eisen, Blei und Silber auch noch die Metalle Quecksilber, Zink und Gold auf.

Bevorzugt bestehen die Gongs insbesondere aus Bronze. Der Tam-Tam-Gong wird beispielsweise über einen sehr langen Zeitraum (eine Woche) von mehreren Personen hergestellt. Die grobe Form wird aus Bronze gegossen und kommt ca. 150 Mal ins Schmiedefeuer.

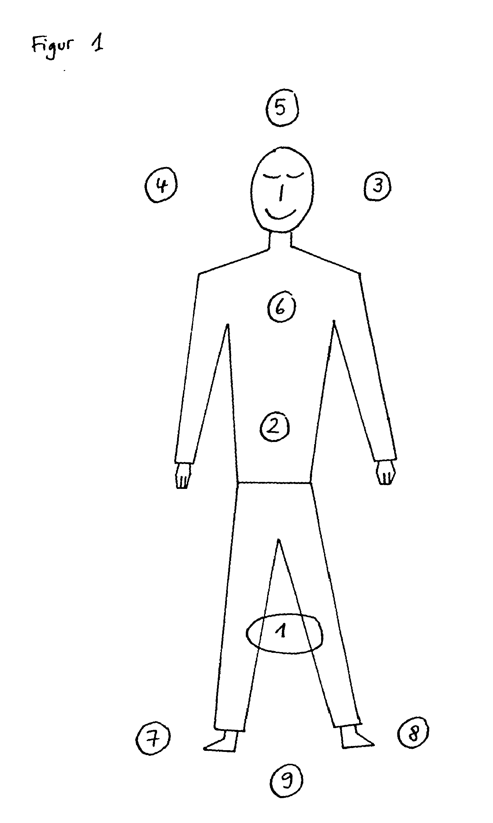

Die Anordnung der Klangschalen erfolgt insbesondere so, dass entweder der Kopf, das Herz, der Bauch, die Knie oder die Füße mit den Klangschalen in Kontakt gebracht werden, oder aber, dass die Klangschalen in der Nähe dieser angeordnet werden. Die Anordnung der Klangschalen erfolgt nach dem Energiefeld des Menschen. In der alternativen Medizin ist dies auch unter dem Begriff "Blume des Lebens" bekannt (siehe Fig. 1). In der Nähe des Kopfes wird insbesondere ein Tam-Gong und am Fußende insbesondere ein Feng-Gong eingesetzt. Am Kopf können insbesondere Kopfschalen angebracht werden, die um den Kopf herum positioniert werden. In einer bevorzugten Form handelt es sich um drei Schalen, die in Form eines Dreiecks in einem gewissen Abstand um den Kopf auf dem Boden bei einem liegenden Patienten positioniert sind. Beim liegenden Patienten kann dann direkt oder in der Nähe des Herzens eine Herzschale, auf dem Bauch oder in der Nähe des Bauches eine Bauchschale und/oder eine Knieschale auf den Knien abgestellt werden. Die Fußschalen können analog zu den Kopfschalen um die Füße herum, bevorzugt in Form eines Dreiecks aufgestellt werden. Bevorzugt können die Kopfschalen und die Fußschalen so aufgestellt werden, dass sie - wenn sie die Form eines Dreiecks einnehmen - jeweils mit der Dreiecksspitze auf die Tam-Gong- bzw. Feng-Gong-Schale, die in einem gewissen Abstand aufgestellt werden, positioniert vorliegen.

Der Fachmann kann in Abhängigkeit von dem Alter oder dem Geschlecht des Patienten die erfindungsgemäße Lehre variieren. Weiterhin kann der Fachmann, ohne selbst erfinderische tätig zu werden, in Abhängigkeit dem zu erreichenden Ziel bestimmen, ob er das sauerstoffhaltige Gasgemisch vor, während und/oder nach der Einwirkung der Schallwellen appliziert. Besonders bevorzugt ist, dass das sauerstoffhaltige Gasgemisch während der Einwirkung der Klangschalen appliziert wird.

In einer weiteren bevorzugten Ausführungsform der Erfindung werden Klangschalen aus Messing eingesetzt; in einer besonders bevorzugten Variante aus einer Zinn-Kupfer Legierung, wobei der Zinnanteil bevorzugt zwischen 25 und 40 % liegt und die Wanddicke der Klangschalen 0,5 bis 5 mm beträgt. Es war völlig überraschend, dass Klangschalen aus Zinn-Kupfer Legierungen und der genannten Wanddicke besonders gut und effizient für die erfindungsgemäße Klang-/Sauerstoff-Therapie eingesetzt werden können.

In einer anderen bevorzugten Ausführungsform des Verfahrens erfolgt die Bereitstellung des Gasgemisches mit einem Sauerstoffanteil von über 90% Vol. durch einen Sauerstoffkonzentrator. Der Sauerstoffkonzentrator ermöglicht es auf eine effiziente und sichere Weise die entsprechenden Sauerstoffkonzentrationen bereit zu stellen.

In einer weiteren bevorzugten Ausführungsform der Erfindung weist der Sauerstoffkonzentrator eine Flussrate von 1 bis 4 Liter pro Minute bei einer Sauerstoffkonzentration von bis zu 95% Vol. +/-3% Vol. auf. Es war besonders überraschend, dass bei diesen Sauerstoffkonzentrationen Stresszuständen mit erhöhtem Sauerstoffverbrauch, sowie Migräne oder Asthma bronchiale besonders gut entgegen gewirkt werden können. Weiterhin kann die Erholung nach einer Krankheit bzw. deren Therapie beschleunigt und verbessert werden.

In einer anderen bevorzugten Ausführungsform ist vorgesehen, dass die Bereitstellung von reinem Sauerstoff durch den Einsatz eines Inhalationsgerätes erfolgt, dass an einem mit reinem Sauerstoff gefüllte Gasflasche angeschlossen ist.

Die genannten Vorzugsvarianten eignen sich überraschend gut, um die Leistungsfähigkeit eines Patienten zu erhöhen. Weiterhin werden mit dem erfindungsgemäßen Verfahren die Vitalität und die Selbstheilungskräfte auf natürliche Weise ohne Nebenwirkungen aktiviert. Durch das erfindungsgemäße Verfahren kann Sauerstoff im Körper besser verteilt werden und jede Zell kann optimal mit Sauerstoff versorgt werden. Daher ist das erfindungsgemäße Verfahren sehr gut zum Stressabbau, zur Verbesserung des Gedächtnisses und der Konzentration aber auch zur Verbesserung der körpereigenen Abwehr einsetzbar. Weitere Bereiche, in welchem das erfindungsgemäße Verfahren eingesetzt werden kann, zum Beispiel die Verbesserung der Kreislaufsituation oder die Krebsnachbehandlung. Das Verfahren kann weiterhin unterstützend bei der Behandlung von Depressionen aber auch bei der Leberentgiftung oder bei der Verringerung der Zellübersäuerung eingesetzt werden.

Die erfindungsgemäße Lehre kann auf alle auf energetischer Basis beruhenden Therapien mit Sauerstoff erweitert werden. Der Vorteil bei dieser Erweiterung auf andere, auf energetischer Basis beruhenden Therapien ist das unkomplizierte Arbeiten ohne manuellen Zeitaufwand, wie es die Klangtherapie erforderlich macht.

### Prophylaktische Aspekte der Erfindung:

Um vorzeitiger Alterung und Ermüdungserscheinungen vorzubeugen (Zellentsäuerung, Entgiftung der Zellen, Zellen in ihrer normalen Schwingung halten um körperliches und seelisches Gleichgewicht zu halten)

### Kosmetische Aspekte:

- Hautbild wird positiv beeinflusst (Zellerneuerung)
- Haarwuchs gefördert
- Cellulitis entgegengewirkt
- Ausstrahlung wird verstärkt (Energetisierung)

Die Therapie sollte immer individuell auf den Patienten abgestimmt und besprochen werden. Der Zustand des Patienten, der Klang und die Resonanz der Klanginstrumente bestimmen den zeitlichen Abstand der zwischen den einzelnen Anwendungen der Sauerstoffklangtherapie liegen sollte. Die energetische Wirkung der Klanginstrumente beträgt ca. 3 Tage. Man sollte die Therapie allerdings nicht über Wochen verteilen, da sonst die Schwingungen wieder neu aufgebaut werden müssen. Angewandt bei:
- Hyperaktivität bei Kindern und Erwachsenen
- leichtere Entbindung bei Schwangeren
- Blutdruck ist sehr gut zu beeinflussen
- Suchtbekämpfung (Patient kann Belastendes leichter verarbeiten und loslassen)
- Beschwerden der Skelettmuskulatur (z.B. Muskelverspannungen, Muskelzerrung).

Unter Zufuhr von 94,5% 02 über einen Konzentrator mit Nasenbrille erhält der Patient die Klangtherapie (Dauer ohne Vorbehandlung ca. 45 Min.). Diese Kombination macht es möglich, alle Zellen optimal mit Sauerstoff zu versorgen, was mit bisherigen Verfahren nicht ausreichend der Fall war. Die Schwingungen der Klangschalen, Gongs, Zimbel und Glocke, welche nach einem ganz bestimmten Ablauf gespielt werden, versetzen jede Zelle in Schwingungen und so kann der 02 optimal aufgenommen werden. Die damit verbundenen Erfolge sind bahnbrechend und ohne jegliche Nebenwirkungen in allen medizinischen Bereichen einsetzbar. Psychische und körperliche

Beschwerden sind so auf ganz natürliche Art behandelbar und ohne Nebenwirkung.

### Wirkung der einzelnen klangerzeugenden Instrumente

TAM - GONG (Bronze aus CHINA)

Gibt ein Gefühl des Getragenseins, des Loslassens. Regt das Immunsystem an. Befreit von Besetzungen (Schizophrenie). Setzt Heilungsprozesse in Gang. Lösung von Traumata, Reinigung der Aura, Verstärkung der Schwingungen im Körper

### FENG - GONG

Der Feng-Gong erzeugt einen schwebenden Klang. Dieser hilft dabei körperliche und seelische Anspannung loszulassen.

### MONOCHORD:

Wird zum Ende und zum Beginn der KT angespielt und hat eine harmonisierende Wirkung durch den OM-Ton.

### KLANGSCHALEN:

Bevorzugt tibetanische Klangschalen in unterschiedlichen Tonhöhen und Größen.
Hohe Tonhöhen im Kopfbereich, tiefere im Fußbereich. Kleine KS kommt auf die Herzgegend, große Klangschale auf die Knie, große, nach innen schwingende Klangschale auf den Bauch (Schwingungen sprechen so intensiver die Bauchorgane an).
Bei Bedarf Vorarbeit zum Auflockern der jeweiligen Körperregion ebenfalls mit Bauchschale, zusätzlich mit warmen Wasser gefüllt um die Schwingungen noch zu verstärken. Die unterschiedlichen Körperfrequenzen werden über die unterschiedlichen Frequenzen der Klangschalen in ihre natürliche Schwingung gebracht. Körperzellen werden auf diese Art gereinigt, regeneriert und erneuert. Der Energiefluss wird angeregt und somit können Blockaden aufgelöst werden. Der Körper wird harmonisiert und findet so auf psychischer und körperlicher Ebene Heilung. Die Schwingungen der KS sind mit einem Ultraschallgerät zu vergleichen was die Reinigung der Zellen betrifft.

Unnatürliche Energiequellen greifen in das natürliche Energiefeld des Menschen ein und können auf keinen Fall diese Schwingungen ersetzten.

### ZIMBEL:

Die hohen Schwingungen der Zimbel ermöglichen die Energiezentren (Chakren), welche in Organverbindungen stehen zu aktivieren.

### GLOCKE:

Die Glocke wird über den Scheitelchakra hochgerieben, angestaute Energien im Kopfbereich können so über den Körper ausgeleitet werden oder bei Energiemangel angeregt werden.(Kopf wird freier, klarer)

Im folgenden soll das Verfahren anhand von Beispielen näher erläutert werden ohne auf diese Beispiele beschränkt zu sein.

Patienten welche Monate unter Verspannungen, Verkrampfungen und Muskelzerrungen litten und durch physiotherapeutische Maßnahmen keine Verbesserungen spürten, fanden bei meiner S-K-T rasche Hilfe.

### Fallbeispiele:

weibliche Patientin: 47 Jahre
Diagnose: Schizophrenie
Invalidenrentnerin

Patientin erhält SKT im Abstand von 3-4 Wochen. 1 Stunde 02 dazu Ablauf KT ca. 45 Minuten. Patientin wird aktiver nach der 4. Behandlung. Denkleistungen, Sprache werden schneller und depressive Verhaltensweise löst sich ganz auf. Die Familie ist über diesen Erfolg erstaunt, da Ihre Motorik deutliche Verbesserungen aufzeigt und der Antrieb etwas aktiver am Leben teilzunehmen spürbar zunimmt.
weibliche Patientin: 76 Jahre
Rentnerin
Beschwerden: starke Gehstörungen
Bauchbeschwerden seit dem 35. Lebensjahr (psychisches Problem, da kinderlos) ständige Übelkeit, Bluthochdruck
Pat. erhält wöchentlich 1 SKT ,( 1 Stunde 02 und 45 Min. KT Ablauf.)

Deutliche Besserung schon nach der 3. SKT. Patientin läuft besser. Patientin erhält auf Wunsch nach wöchentlicher Behandlung im 14-tägigen Abstand 1 SKT um diesen Zustand auch weiterhin zu erhalten. Sie kann wieder allein in die Stadt und sogar am Seniorensport kann Sie wieder teilnehmen. Blutdruck wird reguliert und die Patientin ist wieder aktiver und fühlt sich deutlich besser.
weibliche Patientin : 52 Jahre
Bankangestellte
Diagnose: Diabetes, Bluthochdruck, psychische Anspannung mit starken Rückenbeschwerden
Therapie: 1 Stunde 02 mit Klangtherapie ca. 45 Min und Vorarbeit

Nach der 1. Therapie deutliche Verbesserung des Seelenzustandes, starke Erleichterung in seelischen wie im körperlichen Befinden. Blutdruck ist stabilisiert und die Patientin fühlt sich erholt und ausgeglichen.
weibliche Patientin: 54 Jahre
Geschäftsfrau
Diagnose: starke psychische Anspannung mit Gewichtsverlust
Therapie: 1 Stunde 02 und 45 Min. KT nach Ablauf
Patientin fühlt sich schon nach der 3 SKT deutlich kräftiger, so dass sie wieder voll ins Geschäftsleben einsteigen kann.

Eine regelmäßige Wiederholung der SKT in größeren Abständen, um die Zellen in Ihrer Schwingung zu halten und die Zellen mit genügend Sauerstoff zu versorgen ist empfehlenswert, aber nicht erforderlich.

Die folgenden Beispiele verdeutlichen die Anwendung der erfindungsgemäßen Lehre zunächst in Bauchlage (siehe Figur 1).
1. Tam-Gong und Feng Gong 1x anschlagen
2. Glocke am Wurzelchakra hochreiben, läuten, über Wirbelsäule hochziehen und am 3. Auge seitlich rausziehen. Zurückziehen, über Wirbelsäule ziehen und an den Füßen rausgehen.
3. Knieschale aufsetzen - anschlagen mit Leder-Holzklöppel
   Bauchschale aufsetzen - anschlagen mit Leder- Holzklöppel Knie- und Bauchschale im Wechsel anschlagen ca. 10x
4. Klöppel wechseln (ab jetzt immer kleinen Filzklöppel zum Anschlagen benutzen)
   Herzschale aufnehmen
5.Runde:
   Knie - Bauchschale anschlagen, links- rechts -Kopf oben - Herzschale anschlagen, rechts - links- Fußschale unten anschlagen
6. Harmonierunde:
   Runde wie oben Runde 5, ca. 10x
7. Knie - Bauchschale anschlagen, Zimbel vom Wurzelchakra zum Genick, Knie - Bauchschale anschlagen und Runde
8.Knie - Bauch anschlagen - Zimbel vom Wurzelchakra zu den Füßen, anschließend Runde
9.Knie - Bauchschale anschlagen - Zimbel vom Wurzelchakra zur linken und rechten Niere oder Handchakren anschlagen. Runde
10. Harmonierunde, ca. 10x
11. Knie - Bauchschale anschlagen 2x, Zimbel und Monochord spielen, anschließend Herz-, Knie- und Bauchschale weg

### Patient bzw. Klient geht in die Rückenlage

Ein folgendes weiteres Beispiel illustriert die erfindungsgemäße Lehre mit einem Patienten in Rückenlage.
1. Zimbel - Monochord (wenn vorhanden) Zimbel anschlagen Ticka: (linke Hand weggestreckt, rechter Mittelfinger leicht zwischen die Augenbrauen aufdrücken) ca. 1 Sekunde
2. Tam-Gong und Feng-Gong anschlagen 1x
3. Glocke reiben über Kronenchakra, über Kronenchakra hochziehen über den Körper runterziehen, am Wurzelchakra läuten und über den Beinen die liegende Acht ziehen. Glocke über den Füßen raus.
4. Knie- und Bauchschale aufsetzen und anschlagen mit Leder-Holzklöppel ca. 10x
5. Klöppel wechseln und Herzschale aufnehmen (Filzklöppel)
6. Knie - Bauchschale anschlagen, Kopfschalen anschlagen, Herzschale aufsetzen und anschlagen, Fußschalen anschlagen
7. Harmonierunde ca. 10x
8. Knie - Bauchschale anschlagen - Zimbel über Wurzelchakra läuten
   RUNDE
   Knie - Bauch - Zimbel über Sakralchakra läuten - RUNDE
   Knie - Bauch - Zimbel über Manipurachakra läuten -RUNDE
   Knie - Bauch - Zimbel über Herzchakra läuten - RUNDE
   Knie - Bauch - Zimbel über Halschakra läuten - RUNDE
   Knie - Bauch - Zimbel über dem 3. Auge läuten - RUNDE
   Knie - Bauch - Zimbel über Kronenchakra läuten - RUNDE Zimbel über Kronenchakra erneut anschlagen und Zimbel im Bogen zum Wurzelchakra und im Bogen zu den Füßen ziehen.
9. Schnelle RUNDE ca. 10x
10. Knie - Bauch TAM-Gong anschlagen Runde wie gehabt, ohne Knie und Bauchschale - FENG-Gong anschlagen.
   Diese RUNDEN 4 x anschlagen dabei TAM-Gong wie folgt anschlagen:
   1. Runde
   2. Runde
   3. Runde
   4. Runde
11. Knie - Bauch - Tam-Gong 2x anschlagen Kopf - Herz Fußschalen anschlagen - FENG-Gong 2x anschlagen - Knieschale weg
12. TAM-Gong 2x anschlagen, Kopf - Herz - Fußschalen anschlagen, FENG-Gong 2x anschlagen - Bauchschale weg
13. TAM-GONG kreativ spielen. RUNDE ohne Knie und Bauchschale spielen. FENG-Gong kreativ spielen 1x diesen Ablauf wiederholen (Herzschale weg)
14. TAM-Gong zum Schwingen bringen anschließend, FENG-Gong zum Schwingen bringen (ohne Schalen)
15. Handgong 3 x von innen nach außen anschlagen und 3x von außen nach innen anschlagen
16. Handgong zur rechten Kopfseite und zur linken Seite 1x anschlagen
17. Tunnel: TAM-Gong und Handgong im Wechsel anschlagen
18. Handgong anschlagen und um den Klienten herumlaufen, Feng-Gong und Handgong anschlagen, Spirale über den Füßen ausführen (nicht im Kopfbereich!!)
19. Handgong aufhängen und mehrmals anschlagen, TAM-Gong und FENG-Gong bespielen
20. Zimbel mehrmals läuten und Monochord (wenn vorhanden) spielen. Anschließend ein Musikstück spielen.

## Patentansprüche

1. Verfahren zur Entspannung und zur Reduzierung von Ermüdungserscheinungen, **dadurch gekennzeichnet, dass** Schallwellen durch klangerzeugende Elemente auf den Körper der zu behandelnden Person appliziert werden und zusätzlich reiner Sauerstoff oder ein Gasgemisch mit einem Sauerstoffanteil von über 90% Vol. mittels einer geeigneten Vorrichtung wie beispielsweise einer Atemmaske appliziert wird.

2. Verfahren nach Anspruch 1 **dadurch gekennzeichnet, dass** der Sauerstoff oder das Gasgemisch mit einem Sauerstoffanteil von über 90% Vol. vor und/oder während und/oder nach der Schallwellenapplikation verabreicht wird.

3. Verfahren nach einem der vorhergehenden Ansprüche, **dadurch gekennzeichnet, dass** als Elemente zur Klangerzeugung Klangschalen und/oder Gongs und/oder Zimbeln und/oder Glocken zum Einsatz kommen.

4. Verfahren nach einem der vorhergehenden Ansprüche, **dadurch gekennzeichnet, dass** zur Klangerzeugung Klangschalen aus Messing, in einer bevorzugten Variante aus einer Zinn-Kupfer-Legierung zum Einsatz kommen, wobei der Zinnanteil bevorzugt zwischen 25% und 40% liegt und die Wanddicke der Klangschalen zwischen 0,5 mm und 5 mm beträgt.

5. Verfahren nach einem der vorhergehenden Ansprüche, **dadurch gekennzeichnet, dass** die Bereitstellung des Gasgemisches mit einem Sauerstoffanteil von über 90% Vol. durch den Einsatz eines O₂-konzentrators erfolgt.

6. Verfahren nach einem der vorhergehenden Ansprüche, **dadurch gekennzeichnet, dass** der O₂-konzentrator eine Flussrate von 1 bis 4 Liter pro Minute bei einer O₂-Konzentration von bis zu 95% Vol. +/-3% Vol. aufweist.

7. Verfahren nach einem der vorhergehenden Ansprüche, **dadurch gekennzeichnet, dass** die Bereitstellung von reinem Sauerstoff durch den Einsatz eines Inhalationsgerätes erfolgt, an das eine mit reinem Sauerstoff befüllte Gasflasche angeschlossen ist.
